## Europäisches Patentamt

## European Patent Office

## Office européen des brevets

(19)

(11) Veröffentlichungsnummer: **0 011 078**
**B1**

(12) # EUROPÄISCHE PATENTSCHRIFT

(45) Veröffentlichungstag der Patentschrift:
**19.01.83**

(51) Int. Cl.³: **C 07 D 471/22,** C 09 B 57/00 //
(C07D471/22, 235/00, 221/00)

(21) Anmeldenummer: **79100639.8**

(22) Anmeldetag: **05.03.79**

(54) **Verfahren zur Herstellung der reinen Natriumhydroxid-Additionsverbindungen von cis-Isomeren von Naphthoylen-bis-benzimidazolen.**

(30) Priorität: **08.03.78 DE 2809877**

(43) Veröffentlichungstag der Anmeldung:
**28.05.80 Patentblatt 80/11**

(45) Bekanntmachung des Hinweises auf die Patenterteilung:
**19.01.83 Patentblatt 83/3**

(84) Benannte Vertragsstaaten:
**BE CH DE FR**

(56) Entgegenhaltungen:
**DE-A-1 569 736**
**FR-A-2 104 116**
**US-A-1 927 928**

(73) Patentinhaber: **HOECHST AKTIENGESELLSCHAFT,**
**Postfach 80 03 20, D-6230 Frankfurt/Main 80 (DE)**

(72) Erfinder: **Landler, Josef, Staufenstrasse 40a,**
**D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur Herstellung der reinen Natriumhydroxid-Additionsverbindungen von cis-Isomeren von Naphthoylen-bis-benzimidazolen

Die Erfindung betrifft ein Verfahren zur Herstellung der reinen Natriumhydroxid-Additionsverbindungen der cis-Isomeren von Naphthoylen-bis-benzimidazolen aus wäßrig-alkoholischer alkalischer Lösung unter weitgehender Abtrennung des Alkohols, Behandlung des Rückstands in wäßrig-alkalischer Lösung mit Chlorgas oder Chlorbleichlauge bei Temperaturen bis 90°C, Abtrennung der unlöslichen Nebenprodukte und Fällen der Additionsverbindung aus dem Filtrat, das dadurch gekennzeichnet ist, daß die Fällung der Natriumhydroxid-Additionsverbindung durch Zugabe einer Natriumionen abgebenden Verbindung zur heißen Lösung erfolgt.

Aus der DE-C-2 039 465 ist ein Verfahren zur Herstellung von cis-Naphthoylen-bis-benzimidazol in einer besonderen Kristallmodifikation beschrieben, wobei man das rohe cis-Naphthoylen-bis-benzimidazol in Wasser und/oder einem Lösemittel auf Temperaturen zwischen etwa 100 und 250°C erhitzt und das so erhaltene Pigment in üblicher Weise isoliert. Als Ausgangsmaterial für die Kristallphasenumwandlung dient hierbei das cis-Isomere, wie man es nach der Kondensation von 1,4,5,8-Naphthalintetracarbonsäureanhydrid und 1,2-Diaminobenzol in Äthanol und anschließende Isomerentrennung erhält. Hierbei wird die äthanolische Suspension mit Kaliumhydroxid versetzt, wobei das trans-Isomere als Kaliumhydroxid-Additionsprodukt ausfällt und abgetrennt wird. Aus dem Filtrat wird das Äthanol restlos abdestilliert, der Rückstand mit Wasser aufgenommen und bei 60 bis 70°C mit Chlorbleichlauge verrührt. Nach Reinigung mit Aktivkohle und Filtration wird in das Filtrat Trockeneis eingetragen, wobei sich die gelbe Alkaliadditionsverbindung kristallin abscheidet. Hieraus wird, gegebenenfalls nach einer Nachreinigung mit Chlorbleichlauge, das Rohpigment durch Hydrolysieren in Wasser freigesetzt. Dieses Rohprodukt eignet sich jedoch wegen der schlechten Überlackierechtheit und des trüben Farbtons noch nicht als Pigment und wird deshalb durch eine thermische Behandlung in Wasser und/oder einem Lösemittel in eine andere Kristallmodifikation übergeführt.

Demgegenüber wird erfindungsgemäß die Fällung nicht mit Kohlendioxid, sondern durch Zusatz von Natriumionen zum heißen Filtrat bewirkt. Hierbei ist nicht nur ein erneuter Reinigungsschritt, sondern auch die Phasenumwandlung überflüssig, da bereits durch Hydrolyse ein brauchbares Pigment erhalten wird.

Aus der DE-A-567 210 ist es bereits bekannt, daß die bei der Kondensation von Naphthalin-1,4,5,8-tetracarbonsäure mit o-Phenylendiamin erhaltenen cis-trans-Isomeren in ihrer Löslichkeit derart stark voneinander verschieden sind, daß eine einfache Trennung erfolgen kann. Es wird hierbei das Kondensationsprodukt mit Kaliumhydroxid und Äthanol erhitzt, wobei die cis-Verbindung in Lösung geht. Nach Abtrennen der unlöslichen trans-Additionsverbindung wird aus dem Filtrat das cis-Isomere mit Wasser ausgefällt. Es wird auch erwähnt, daß anstelle von Kaliumhydroxid Natriumhydroxid verwendet werden kann.

Aus der DE-A-1 569 736 ist es bekannt, daß man die reinen Isomeren von Naphthoylen-bis-benzimidazolen in einem Arbeitsgang erhalten kann, wenn man Naphthalin-1,4,5,8-tetra-carbonsäure oder deren Anhydride mit 1,2-Diaminobenzol in niedrigsiedenden Alkoholen bei Temperaturen zwischen 120 und 180°C unter Druck kondensiert, das entstandene Isomerengemisch im selben Medium durch Behandlung mit Alkalien in die Alkali-Additionsverbindungen überführt und diese aufgrund ihrer verschiedenen Löslichkeit durch Filtration voneinander trennt. Als Alkalien kommen in erster Linie Natrium- oder Kaliumhydroxid in Frage. Im Beispiel wird erwähnt, daß der aus der alkoholischen Mutter- und Waschlauge erhaltene Farbstoff in reinerer Form anfällt, wenn man zunächst einen Teil des eingesetzten Äthanols aus den vereinigten Mutterlaugen abdestilliert, die ausgefallene Kaliumadditionsverbindung absaugt und anschließend in warmem Wasser hydrolysiert.

Aus der US-A-3 072 661 ist es bekannt, aus den wäßrig-äthanolischen alkalischen Mutterlaugen den Alkohol weitgehend abzudestillieren, bis der Gehalt an Alkohol nur noch etwa 20 Gew.-% beträgt, das abdestillierte Volumen durch Wasser zu ergänzen, die so erhaltene Lösung mit Filtrierhilfsmitteln zu klären, das cis-Isomere mit der 6- bis 12fachen Gewichtsmenge, bezogen auf das cis-Isomere, an p-Toluolsulfonsäure zu fällen und die Suspension anschließend zunächst auf etwa 55°C, dann auf 75 bis 90°C zu erhitzen und anschließend das in pigmentärer Form erhaltene Produkt abzutrennen.

Bei allen diesen bekannten Verfahren befriedigt die Reinheit des erhaltenen Produktes noch nicht. Die in den Produkten enthaltenen Verunreinigungen machen sich hierbei in einer mehr oder weniger starken Abtrübung des Farbtons und auch in einer Verschlechterung der Echtheitseigenschaften, insbesondere der Migrationsechtheiten und vor allem der Lösemittel- und Überlackierechtheit, bemerkbar.

Es wurde gefunden, daß die erfindungsgemäß gereinigten Produkte sowohl beim Einsatz als Küpenfarbstoff auf Baumwolle als auch in Pigmentform den bekannten Produkten überlegen sind. So zeichnen sich die erfindungsgemäß erhaltenen Farbmittel durch einen sehr reinen Farbton, höhere Farbstärke und verbesserte Echtheitseigenschaften aus.

Im folgenden werden einige bevorzugte Ausgestaltungen der Erfindung näher erläutert:

Bei der weitgehenden Abtrennung des Alkohols, im allgemeinen Äthanol, aus den wäßrig-alkoholischen alkalischen Lösungen wird der Alkohol je nach verfügbarer Apparatur in dem Maße zurückgewonnen, wie dies noch wirtschaftlich ist. Da der zurückbleibende Alkohol einen erhöhten Chlorverbrauch erfordert, wobei das hierbei anfallende Chlorid eine Aufarbeitung des Abwassers erfordert, wird der Alkohol zweckmäßig so vollständig wie möglich abdestilliert.

Anstelle einer Behandlung mit Chlorgas kann auch — da im wäßrig-alkalischen Medium gearbeitet wird — die sogenannte »Chlorbleichlauge« eingesetzt werden. Vorzugsweise findet die oxidierende Behandlung bei Temperaturen im Bereich bis 70° C, insbesondere von 20 bis 60° C, statt, wobei zunächst so lange Chlor in dem Maß zugegeben wird, wie es verbraucht wird. Zweckmäßig wird das Reaktionsgemisch mit einem geringen Chlorüberschuß einige Zeit, beispielsweise eine halbe Stunde, bei 55 bis 65° C oder leicht erhöhter Temperatur gerührt und anschließend das Reaktionsgemisch auf erhöhte Temperatur gebracht, wobei das restliche Chlor verbraucht wird.

Die bei der Oxidation entstandenen unlöslichen Produkte werden abgetrennt, zweckmäßig nach Zusatz von Klärhilfsmitteln abfiltriert. Als Klärhilfsmittel kommen beispielsweise Bleicherden und/oder Aktivkohle in Betracht.

Als Natriumionen abgebende Verbindung kommen Natriumhydroxid und/oder Natriumsalze, aus wirtschaftlichen Gründen insbesondere Natriumchlorid oder Natriumsulfat sowie Salzgemische in Betracht. Da die Fällung der im Verhältnis zur Kaliumhydroxid-Verbindung schwerer löslichen Natriumhydroxid-Additionsverbindung beabsichtigt ist, richtet sich die Menge der zugesetzten Natriumionen abgebenden Verbindung nach der bereits vorhandenen Konzentration an Kalium- und Natriumionen. Die zur vollständigen Fällung der Natriumhydroxid-Additionsverbindung erforderliche Natriumionenkonzentration kann leicht durch einen einfachen Vorversuch ermittelt werden. Die Natriumionen abgebende Verbindung wird in das heiße geklärte Filtrat vorzugsweise in fester Form eingebracht, gegebenenfalls auch in einer möglichst konzentrierten Lösung. Die Natriumhydroxid-Additionsverbindung scheidet sich beim Abkühlen in gut kristalliner Form ab.

Sofern die Natriumhydroxid-Additionsverbindung isoliert werden soll, kann sie — je nach der gewünschten Reinheit — noch durch Waschen mit konzentrierter Natronlauge von der anhaftenden Mutterlauge befreit werden. Das so erhaltene Produkt kann dann beispielsweise in eine Pigmentpräparation überführt werden, wobei das Präparationsmittel bereits bei der Freisetzung des Pigmentkorns durch Hydrolyse der Natriumhydroxid-Additionsverbindung zugegen ist.

Es ist auch möglich, die Natriumhydroxid-Additionsverbindung ohne Isolierung unmittelbar mit Wasser oder verdünnter Säure zu hydrolysieren. Je nach der geforderten Reinheit des Produktes kann hierbei die Natriumhydroxid-Additionsverbindung mit konzentrierter Natronlauge gewaschen oder beispielsweise nur weitgehend trockengesaugt werden.

Das erfindungsgemäße Reinigungsverfahren eignet sich nicht nur für das unsubstituierte cis-Naphthoylen-bis-benzimidazol, sondern auch für dessen Derivate, die gegen das erfindungsgemäße oxidative Verfahren hinreichend stabil sind.

In den folgenden Beispielen beziehen sich die Prozentangaben auf das Gewicht.

## Beispiel 1

Das aus der Kondensation von 100 Gewichtsteilen Naphthalin-1,4,5,8-tetracarbonsäureanhydrid mit 76 Gewichtsteilen 1,2-Diaminobenzol erhaltene Isomerengemisch wird zur Trennung der beiden Isomeren gemäß DE-B-1 569 736 mit Kaliumhydroxid in Äthanol-Wasser bei Siedetemperatur behandelt. Nach Bildung der schwerlöslichen KOH-Verbindung des trans-Isomeren wird diese durch Filtration abgeschieden.

Aus dem Filtrat, worin das cis-Isomere enthalten ist, wird das gesamte Äthanol abdestilliert. In den verbleibenden Destillationsrückstand werden unter Rühren, beginnend bei 20° C, 20 Gewichtsteile gasförmiges Chlor eingeleitet, wobei die Temperatur auf 50 bis 60° C ansteigt. Danach läßt man 380 Gewichtsteile heißes Wasser zulaufen und trägt je 6 Gewichtsteile Bleicherde und Aktivkohle ein. Man rührt 30 Minuten bei 90° C nach und filtriert heiß. In das heiße Filtrat werden 80 Gewichtsteile festes Natriumhydroxid eingerührt. Nach dem Lösen wird die Rührung abgestellt und zum Auskristallisieren des reinen cis-Isomeren der NaOH-Additionsverbindung auf 20° C abgekühlt. Dieses wird auf einer Nutsche isoliert und mit 25%iger Natronlauge die anhaftende Mutterlauge ausgewaschen.

## Beispiel 2

Zunächst wird verfahren wie im Beispiel 1 angegeben. Nach Isolation des äthanolfreien Destillationsrückstandes wird die Oxidation an Stelle von Chlor mit 340 Gewichtsteilen Chlorbleichlauge (13% akt. Chlor) durchgeführt, wobei nur 60 Gewichtsteile heißes Wasser zugegeben werden. Die isolierte NaOH-Additionsverbindung, sowie das daraus durch Hydrolyse erhaltene cis-Naphthoylen-bis-benzimidazol, sind mit den Produkten, die nach Beispiel 1 erhältlich sind, identisch.

## Beispiel 3

Oxidation und Abtrennung der schwerlöslichen Verunreinigungen werden wie im Beispiel 1 durchgeführt. Dem heißen klaren Filtrat werden 20 Gew.-Teile Natriumhydroxid zugesetzt und nach dessen Auflösung 60 Gew.-Teile Natriumchlorid (in fester Form oder als 30%ige wäßrige Lösung) eingerührt. Man verfährt dann weiter wie in Beispiel 1 beschrieben. Die isolierte Natriumhydroxid-Additionsverbindung sowie das daraus durch Hydrolyse erhaltene cis-Naphthoylen-bis-benzimidazol weisen dieselbe Qualität wie die nach Beispiel 2 erhaltenen Produkte auf.

### Beispiel 4

Die Oxidation wird wie im Beispiel 2 angegeben durchgeführt. Nach dem Abtrennen der Verunreinigungen und Klärhilfsmittel werden der klaren Lösung 200 Gew.-Teile 30%ige wäßrige Natriumchloridlösung zugesetzt. Es wird dann weiter verfahren wie in Beispiel 1 angegeben.

Die isolierte Natriumhydroxid-Additionsverbindung sowie das daraus durch Hydrolyse erhaltene cis-Naphthoylen-bis-benzimidazol weisen die gleiche Qualität wie die nach Beispiel 2 erhaltenen Produkte auf.

Im folgenden Beispiel wird die Überführung einer Natriumhydroxid-Additionsverbindung in einen Küpenfarbstoff beschrieben:

### Beispiel 5

400 Gew.-Teile der nach Beispiel 1 erhaltenen Natriumhydroxid-Additionsverbindung werden in 2 Liter Wasser eingerührt und zur Erleichterung der Filtration zwei Stunden bei 70 bis 80° C nachgerührt. Der ausgefallene Farbstoff wird auf einer Nutsche isoliert, mit Wasser neutral gewaschen und getrocknet.

Das so erhaltene reine cis-Naphthoylen-bis-benzimidazol zeigt als Küpenfarbstoff auf Baumwolle eine erheblich höhere Farbstärke und Reinheit als ein nach dem Stand der Technik erhaltenes Produkt.

In den folgenden Beispielen wird beschrieben, wie eine Natriumhydroxid-Additionsverbindung in eine Pigmentpräparation überführt werden kann:

### Beispiel 6

455 Gew.-Teile des cis-Isomeren der Natriumhydroxid-Additionsverbindung des Naphthoylen-bis-benzimidazols mit einem Gehalt von 22% Reinpigment werden unter kräftigem Rühren in eine vorbereitete Mischung von 5° C, bestehend aus 2 Liter Wasser, worin eine Lösung von 5 Gew.-Teilen eines Polyäthylenwachses (Molekulargewicht: ca. 2000, nicht emulgierbar, Tropfpunkt: 103 – 107° C, Erstarrungspunkt: 90 – 94° C, Säure- und Verseifungszahl: 0, Dichte bei 20° C: 0,91 – 0,92) in 20 Gew.-Teilen Chlorbenzol gut verrührt wurde, eingetragen. Die Pigmentsuspension wird 3 Stunden bei Raumtemperatur nachgerührt. Anschließend wird die Suspension auf 70 – 75° C erwärmt und bei dieser Temperatur weitere 2¹/₂ bis 3 Stunden nachgerührt. Hiernach wird mit Wasserdampf das Chlorbenzol abdestilliert, das Pigment auf einer Nutsche isoliert, mit Wasser neutral gewaschen und im Umluftschrank bei 80 – 90° C getrocknet.

Ausbeute: 105 Gewichtsteile präpariertes Pigment.

Das kreidig-weich getrocknete Pigment läßt sich leicht aufmahlen. Die vorzüglichen Dispergierungseigenschaften dieser Pigmentpräparation ermöglichen die Verwendung auf einem breiten Anwendungssektor. Ganz deutlich läßt sich in der Polyolefin-Massenfärbung die ausgezeichnete Pigmentverteilung und Farbstärke erkennen.

### Beispiel 7

455 Gew.-Teile des cis-Isomeren der Natriumhydroxid-Additionsverbindung des Naphthoylen-bis-benzimidazols mit einem Gehalt von 22% Reinpigment werden unter kräftigem Rühren in eine vorbereitete Mischung von 5° C, bestehend aus 2 Liter Wasser, worin eine Lösung von 5 Gew.-Teilen des im Beispiel 6 beschriebenen Wachses in 20 Gew.-Teilen Chlorbenzol gut verrührt wurde, eingetragen. Die Pigmentsuspension wird 3 Stunden bei Raumtemperatur nachgerührt. Anschließend wird die Suspension auf 70 – 75° C erwärmt und bei dieser Temperatur weitere 2¹/₂ Stunden nachgerührt. Hiernach wird die Suspension abgesaugt, das an der Präparation anhaftende Chlorbenzol mit Äthanol ausgewaschen und hinterher das Nutschgut mit Wasser neutral gewaschen. Die Trocknung erfolgt im Umluftschrank bei 80 – 90° C. Ausbeute: 105 Gew.-Teile präpariertes Pigment. Das auf diese Weise isolierte Pigment ist mit der nach Beispiel 6 erhaltenen Ware identisch.

## Patentansprüche

1. Verfahren zur Herstellung der reinen Natriumhydroxid-Additionsverbindungen der cis-Isomeren von Naphthoylen-bis-benzimidazolen aus wäßrig-alkoholischer alkalischer Lösung unter weitgehender Abtrennung des Alkohols, Behandlung des Rückstands in wäßrig-alkalischer Lösung mit Chlorgas oder Chlorbleichlauge bei Temperaturen bis 90° C, Abtrennung der unlöslichen Nebenprodukte und Fällen der Additionsverbindung aus dem Filtrat, dadurch gekennzeichnet, daß die Fällung der Natriumhydroxid-Additionsverbindung durch Zugabe einer Natriumionen abgebenden Verbindung zur heißen Lösung erfolgt.

2. Verfahren nach Anspruch 1, dadurch gekennzeichnet, daß der Alkohol so vollständig wie möglich abdestilliert wird.

3. Verfahren nach Anspruch 1 und 2, dadurch gekennzeichnet, daß die Behandlung mit Chlorgas bzw. Chlorbleichlauge bei Temperaturen bis 70° C erfolgt.

4. Verfahren nach Anspruch 1 bis 3, dadurch gekennzeichnet, daß die Behandlung mit Chlorgas bzw. Chlorbleichlauge bei Temperaturen von 20 bis 60° C erfolgt.

5. Verfahren nach Anspruch 1 bis 4, dadurch gekennzeichnet, daß die Abtrennung der unlöslichen Nebenprodukte mit Hilfe von Klärhilfsmitteln erfolgt.

6. Verfahren nach Anspruch 1 bis 5, dadurch gekennzeichnet, daß die Natriumionen abgebende Verbindung Natriumhydroxid ist.

7. Verfahren nach Anspruch 6, dadurch gekennzeichnet, daß das Natriumhydroxid in fester Form eingetragen wird.

8. Verfahren nach Anspruch 1 bis 5, dadurch gekennzeichnet, daß die Natriumionen abgebende Verbindung Natriumchlorid ist.

## Claims

1. A process for obtaining pure sodium hydroxide addition products of the cis-isomers of naphthoylene-bis-benzimidazoles from aqueous-alcoholic alkaline solution, by removing the major part of the alcohol, reacting the remainings in aqueous alkaline solution with chlorine gas or an alkali metal hypochlorite at a temperature up to 90°C, removing insoluble by-products and precipitating the addition compound from the filtrate, which comprises carrying out the precipitation of the sodium hydroxide addition product by adding a compound yielding sodium ions to the hot solution.

2. A process as claimed in claim 1, wherein the alcohol is distilled off as completely as possible.

3. A process as claimed in claim 1 and 2, wherein the chlorine or hypochlorite is added at a temperature up to 70° C.

4. A process as claimed in claim 1 to 3, wherein the chlorine or hypochlorite is added at a temperature of 20 to 60° C.

5. A process as claimed in claim 1 to 4, wherein the insoluble by-products are removed by means of a filter aid.

6. A process as claimed in claim 1 to 5, wherein the compound yielding sodium ions is sodium hydroxide.

7. A process as claimed in claim 6, wherein the sodium hydroxide is added in solid form.

8. A process as claimed in claim 1 to 5 wherein the compound yielding sodium ions is sodium chloride.

## Revendications

1. Procédé pour préparer les composés d'addition d'hydroxyde de sodium purs des isomères cis de naphtoylène-bis-benzimidazoles à partir d'une solution aqueuse-alcoolique alcaline, avec séparation poussée de l'alcool, traitement du résidu dans une solution aqueuse alcaline par du chlore gazeux ou une lessive de blanchiment chlorée, à des températures pouvant aller jusqu'à 90°C, séparation des produits secondaires insolubles et précipitation du composé d'addition dans le filtrat, procédé caractérisé en ce que la précipitation du composé d'addition d'hydroxyde de sodium est effectuée par addition, à la solution chaude, d'un composé fournissant des ions de sodium.

2. Procédé selon la revendication 1, caractérisé en ce que l'alcool est chassé par distillation aussi complètement que possible.

3. Procédé selon l'une des revendications 1 et 2, caractérisé en ce que le traitement par le chlore gazeux ou la solution de blanchiment chlorée est effectué à des températures pouvant aller jusqu'à 70°C.

4. Procédé selon l'une quelconque des revendications 1 à 3, caractérisé en ce que le traitement par le chlore gazeux ou la lessive de blanchiment chlorée est effectué à des températures de 20 à 60°C.

5. Procédé selon l'une quelconque des revendications 1 à 4, caractérisé en ce que la séparation des produits secondaires insolubles est effectuée au moyen d'agents de clarification.

6. Procédé selon l'une quelconque des revendications 1 à 5, caractérisé en ce que le composé fournissant des ions de sodium est l'hydroxyde de sodium.

7. Procédé selon la revendication 6, caractérisé en ce que l'hydroxyde de sodium est introduit à l'état solide.

8. Procédé selon l'une quelconque des revendications 1 à 5, caractérisé en ce que le composé fournissant des ions de sodium est le chlorure de sodium.